# EUROPEAN PATENT APPLICATION

(11) **EP 2 711 360 A1**
(43) Date of publication of application: **26.03.2014**
(21) Application number: 12185099.4
(22) Date of filing: 19.09.2012
(51) Int. Cl.: C07D 213/22, H01G 9/20

(54) **Fluorinated cobalt redox mediator, method for producing the same, and its use in dye-sensitized solar cell**

(71) Applicant: Solvay SA, 1120 Bruxelles (BE)
(72) Inventor: Miyaji, Taichi, Kanagawa-ken, Kanagawa 252-0004 (JP)
(74) Representative: Mross, Stefan P.M.

(57) **Abstract**

The present invention provides a metal complex having the formula : (La)n-M, wherein M is cobalt, La is fluorinated bidentate or fluorinated tridentate ligand, and n is 2 or 3. The fluorinated cobalt complex can be used as redox mediator in dye-sensitized solar cell.

## Description

### TECHNICAL FIELD

The present invention relates to a metal complex comprising fluorinated polypyridine ligand, method for producing the same, its use as redox mediator in photoelectric conversion device, particularly in dye-sensitized solar cell, and the dye-sensitized solar cells comprising the same.

### BACKGROUND OF THE INVENTION

Conventional solar cells convert light into electricity by exploiting the photovoltaic effect that exists at semiconductor junctions. In other words, the commercial solar cells absorb energy from visible light and convert excited charge carriers thereof to electric energy. At present, the main commercial solar cells are silicon-based solar cells. For the silicon-based solar cell, there are shortcomings in that high energy costs for material processing is required and many problems to be addressed such as environmental burdens and cost and material supply limitations are involved. For an amorphous silicon solar cell, there are also shortcomings in that energy conversion efficiency decreases when used for a long time, due to deterioration in a short period.

One of the low-cost organic solar cells is a dye-sensitized solar cell (DSSC) which is formed based on a semiconductor sensitized by a dye to absorb incoming light to produce excited electrons, an electrode connected to the semiconductor, a counter electrode and an electrolyte usually comprising the triiodide/iodide (I₃⁻/I⁻) redox mediator.

The triiodide/iodide redox mediator has several drawbacks from both scientific and commercial point of views. US 7,019,138 B2 discloses certain cobalt complexes below as a candidate of alternative redox mediator.

| | | |
|---|---|---|
| 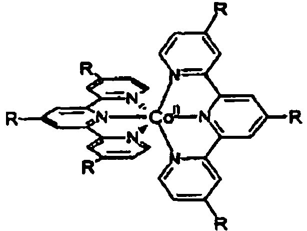 | 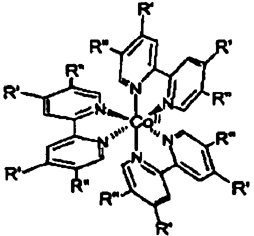 | 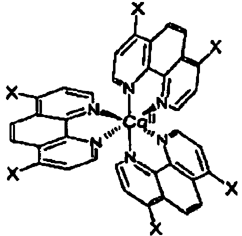 |
| R = ethyl (te-terpy) | | R' = t-butyl, R" = H (dtb-bpy) |
| R = t-butyl (ttb-terpy) | | R' = COO-*t*-butyl, R" = H (dtb-est) |
| R' = H, R" = H (bpy) | | R' = phenyl, R" = H (dp-bpy) |
| R' = CH₃, R" = H (4,4'-dmb) | | R' = 3-pentyl, R" = H (d3p-bpy) |
| R' = H, R" = CH₃ (5,5'-dmb) | | R' = nonyl, R" = H (dn-bpy) |
| R' = CH₃, R" = CH₃ (tm-bpy) | | X = H (phen) |
| 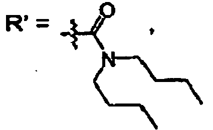 | R' = H (bdb-amd) | X = phenyl (phen-phen) |

### DESCRIPTION OF THE INVENTION

The purpose of the present invention is to provide a metal complex having advantages when used as redox mediator in photoelectric conversion device, especially in dye-sensitized solar cell.

The present invention therefore relates to a metal complex having the formula (I) below :
(La)n-M (I)
   wherein M is cobalt, La is independently selected from the group consisting of the following bidentate and tridentate ligands, and n is 2 or 3 : 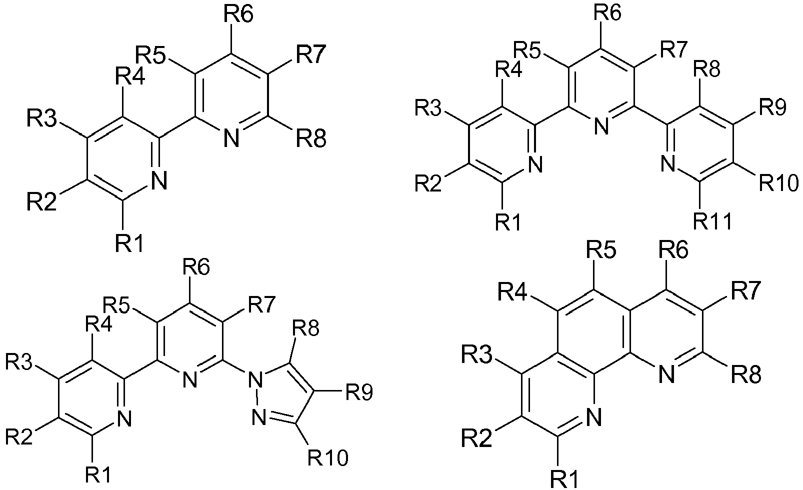 wherein R1, R2, R3, R4, R5, R6, R7, R8, R9, R10 and R11 are independently selected from the group consisting of hydrogen, fluorine, alkyl groups, and fluorinated alkyl groups ; provided at least La comprises at least one fluorine atom. Preferably, R1, R2, R3, R4, R5, R6, R7, R8, R9, R10 and R11 are independently selected from the group consisting of hydrogen, fluorine, CH₃, and CF₃; provided at least La comprises at least one fluorine atom.

It has been surprisingly found that the metal complex according to the present invention provides more positive redox potential without having bulky substituents on the ligand. This is particularly advantageous as the bulky substituents cause retardation of the mobility. The positive shift of redox potential leads to larger photovoltage. Also, the metal complex according to the present invention allows easy fine tuning of the redox potential in accordance to HOMO level of the dye to be used by changing number and position of fluorides or fluorinated substituents on the ligand. Additionally, the metal complex according to the present invention is advantageous from its hydrophobicity and stable characteristics.

In the present invention, "redox mediator" is understood to denote in particular molecules or ions which mediate electron transfer from an electrode to an oxidized dye by their reduction-oxidation properties. More particularly, such redox mediators can be reversibly reduced at the electrode and reversibly oxidized by the oxidized dye.

In the present invention, "alkyl groups" is understood to denote in particular a straight chain, branched chain, or cyclic hydrocarbon groups usually having from 1 to 20 carbon atoms, preferably having from 1 to 8 carbon atoms. Examples of alkyl groups include methyl, ethyl, propyl, isopropyl, butyl, isobutyl, sec-butyl, tert-butyl, pentyl, neopentyl, hexyl, cyclopropyl, cyclobutyl, cyclopentyl, and cyclohexyl. In sense of the present invention, the straight chain hydrocarbon groups having small number of carbon atoms are especially preferred, such as methyl and ethyl, further particularly methyl.

In the present invention, "fluorinated" is understood to denote in particular the fact that at least one of the hydrogen atoms of the respective chemical group has been replaced by a fluorine atom. If all of the hydrogen atoms have been replaced by fluorine atoms, the fluorinated chemical group is perfluorinated. For instance, "fluorinated alkyl groups" include (per)fluorinated alkyl groups such as (per)fluorinated methyl, ethyl, propyl, isopropyl, butyl, isobutyl, *sec*-butyl or *tert-*butyl; and for instance -CF₃, -C₂F₅, heptafluoroisopropyl (-CF(CF₃)₂), hexafluoroisopropyl (-CH(CF₃)₂) or -CF₂(CF₂)₄CF₃.

In one embodiment, La in the formula (I) is selected from polypyridines (for example, bi- and ter-pyridines) comprising at least one fluorine atom, preferably from the group consisting of the following ligands :

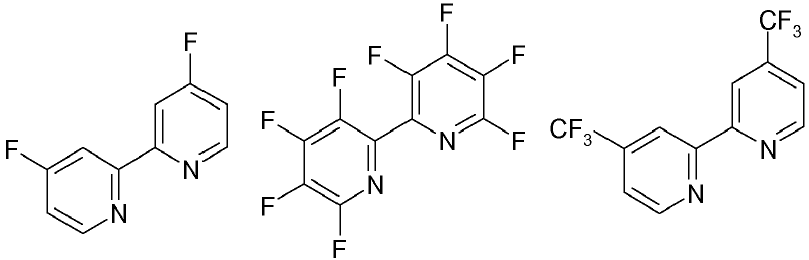

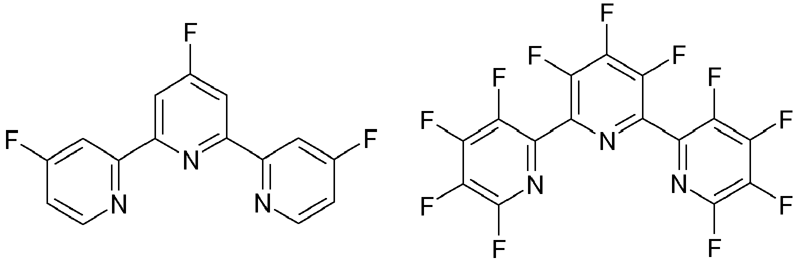

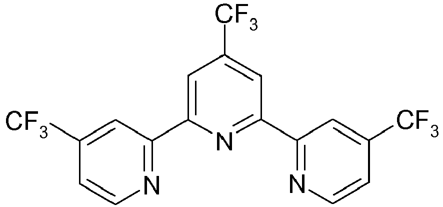

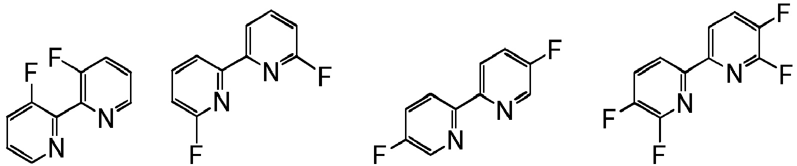

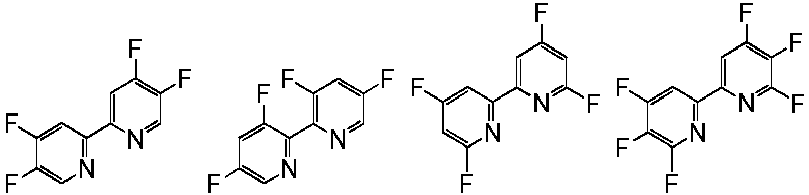

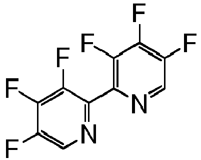

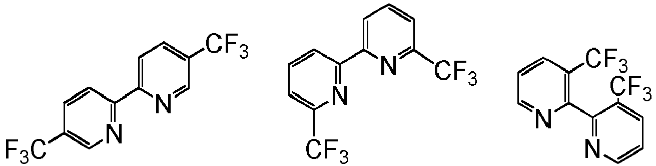

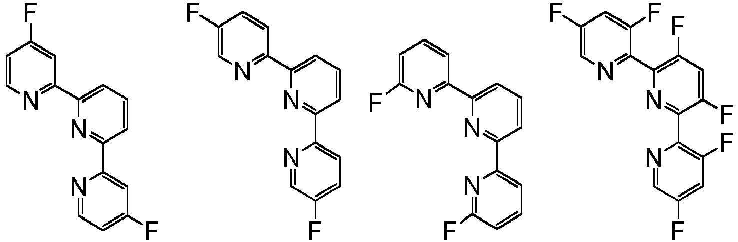

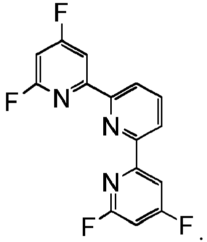

More preferably, La in formula (I) is selected from the group consisting of the following ligands :

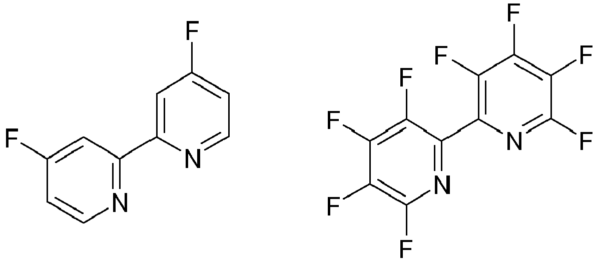

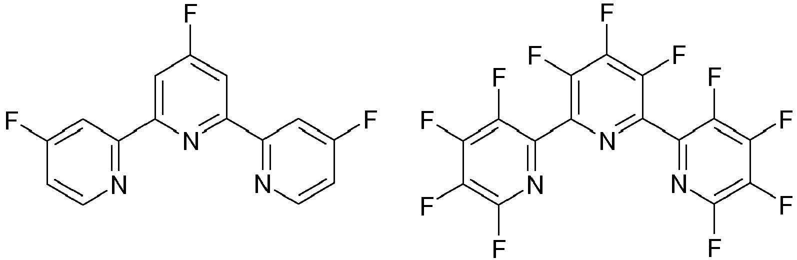

In another embodiment, the metal complex further comprises at least one counter-anion (X). Preferably, the counter-anion (X) is independently selected from monoanions. The number of the counter-anion can be two or three depending on the oxidation state of center metal, i.e. cobalt.

In a further embodiment, the counter-anion (X) is selected from the group consisting of Cl-, Br-, I-, CN-, NCO-, NCS-, NCSe-, amino groups, phosphate groups, PF6⁻, borates such as [BF₄]⁻, tetracyanoborate ([B(CN)₄]⁻), [B(Ph)₄]⁻, [B(C₆F₅)₄]⁻, bis(trifluoromethanesulfonyl)imide (TFSI⁻), bis(fluorosulfonyl)imide (FSI⁻), and any combination thereof, preferably TFSI⁻.

Especially preferred metal complexes according to the present invention are as follows :

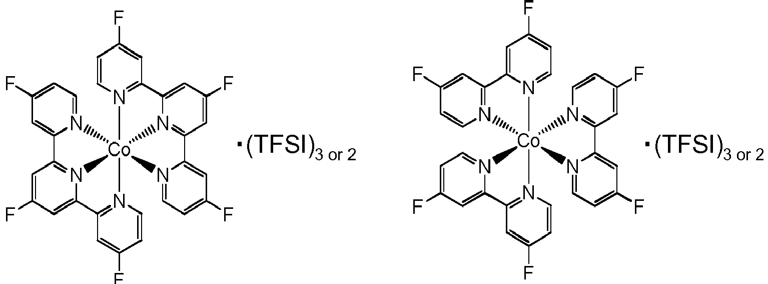

Usually the two complexes, each comprising Co^{II} and Cₒ^{III} (= Co²⁺ and Co³⁺), are used as a mixture when an electrolyte comprising the metal complex according to the present invention as redox mediator is formulated. In the sense of the present invention, Co^{II} is generally major portion.

The metal complexes according to the present invention described herein are suitable for use in photoelectric conversion device, particularly in dye-sensitized solar cell. Therefore, the present invention also relates to a use of the metal complex according to the present invention as redox mediator in photoelectric conversion device, in particular in dye-sensitized solar cell or organic photovoltaic device.

Another advantage of the fluorinated metal complexes according to the present invention is their versatility in regards to the dyes. The better performance in combination with conventional dyes and semiconductors, such as TiO₂ than the triiodide/iodide redox mediator or even other cobalt complexes is achievable by using the metal complexes according to the present invention.

Further, the present invention also concerns a use of cobalt complex having polypyridine ligand comprising at least one fluorine atom, as redox mediator in photoelectric conversion device, in particular in dye-sensitized solar cell.

The basic element of a DSSC is generally a TiO₂ (titanium dioxide) nanoparticulate structure sensitized with dye molecules to form the core of a DSSC. Generally, the dye molecules can be metal complexes, such as ruthenium complexes, organic dyes having D-π-A structure (wherein D is donor groups is bridge group and A is acceptor group), or macrocyclic dyes, such as porphyrin dyes, and phthalocyanine dyes. The assembly of titanium dioxide nanoparticles is well connected to their neighbors. TiO₂ is the preferred material for the nanoparticles since its surface is highly resistant to the continued electron transfer. However, TiO₂ only absorbs a small fraction of the solar photons (those in the UV). The dye molecules attached to the semiconductor surface are used to harvest a great portion of the solar light. Another key component of the DSSC is an electrolyte which transports positive charge carriers from the sensitizing dye to the back contact of the device. Electrolytes containing the iodide/triiodide redox system are most commonly used. However, the iodide/triiodide redox system suffers from the disadvantages, such as low redox potential, corrosive characteristic to metals, and competition with the sensitizing dye in absorbing light.

In this context, it has been found that the metal complexes according to the present invention work in DSSC as redox mediator having improved characteristics, as set forth above, compared to traditional iodide/triiodide redox system and to non-fluorinated cobalt complexes.

Accordingly, the present invention further relates to a dye-sensitized solar cell (DSSC), which comprises the metal complex according to the present invention as redox mediator.

In a further specific embodiment, the DSSC according to the present invention further comprises TiOF₂ as semiconductor. TiOF₂ has the conduction band level at lower energy compared to TiO₂ which should improve the photo-induced electron transfer from the excited dye to the semiconductor. The TiOF₂ is preferably used in the form of TiOF₂ nanoparticles, in particular TiOF₂ particles having a mean primary particle size from 15 to 50 nm. The layer thickness is typically from 500 nm to 10 µm. The TiOF₂ may be used as the sole semiconductor in the DSSC semiconductor layer or may be combined in mixture with any other suitable semiconductor compound, for instance TiO₂. Another possibility is to have at first a dense TiO₂ layer on the conducting glass followed by a nanoporous TiOF₂ layer. The fluorinated metal complexes according to the present invention can be used in combination with TiOF₂ used as semiconductor in dye sensitized solar cells.

The present invention also concerns a method for preparing the dye-sensitized solar cell, comprising :
a step of providing an electrode connected to dye-sensitized semiconductor and a counter electrode ; and
a step of providing an electrolyte;
wherein said electrolyte comprises the metal complex according to the present invention as redox mediator.

While preferred embodiments of this invention have been shown and described, modifications thereof can be made by one skilled in the art without departing from the spirit or teaching of this invention. The embodiments and examples described herein are exemplary only and are not limiting. Many variations and modifications of systems and methods are possible and are within the scope of the invention. Accordingly, the scope of protection is not limited to the embodiments described herein, but is only limited by the claims that follow, the scope of which shall include all equivalents of the subject matter of the claims.

### Examples

### Example 1 : Preparation of the complex (1)

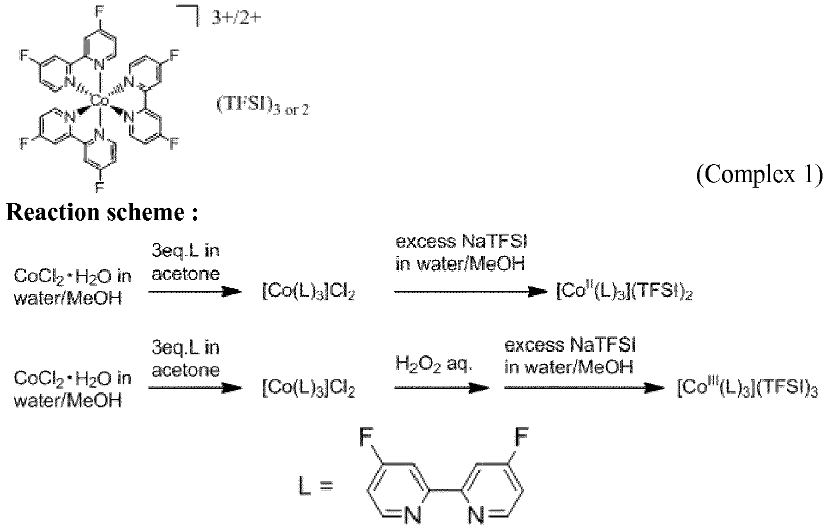

**Preparation of [Co(L)₃](TFSI)₂.** CoCl₂·6H₂O is dissolved in a mixture of water / MeOH. To this is added 3 equiv. L solution in acetone. The mixture is stirred at 50°C for 1 h, followed by addition of NaTFSI (excess) solution in water / MeOH. The mixture is stored overnight at 5°C to aid precipitation. The resulting precipitate is collected by filtration, washed with water and MeOH, and dried *in vacuo.*
**Preparation of [Co(L)₃](TFSI)₃.** To a solution of CoCl₂·6H₂O in water /MeOH is added 3 equiv. L solution in acetone. After stirring for 10 minutes, an aqueous solution of H₂O₂ and HCl is added. The mixture is stirred at 50°C for 1 h, followed by addition of NaTFSI (excess) solution in water / MeOH. The mixture is stored overnight at 5°C to aid precipitation. The resulting precipitate is collected by filtration, washed with water and MeOH, and dried *in vacuo.*

## Claims

1. A metal complex having the formula (I) below :
(La)n-M (I)
wherein M is cobalt, La is independently selected from the group consisting of the following bidentate and tridentate ligands, and n is 2 or 3 : 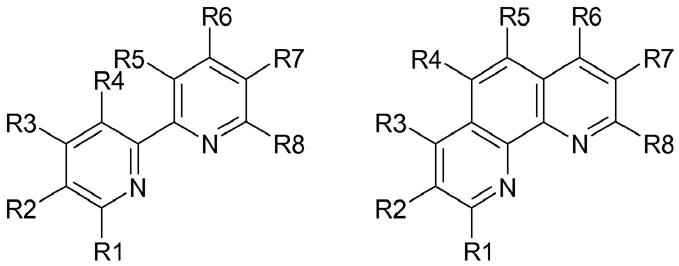 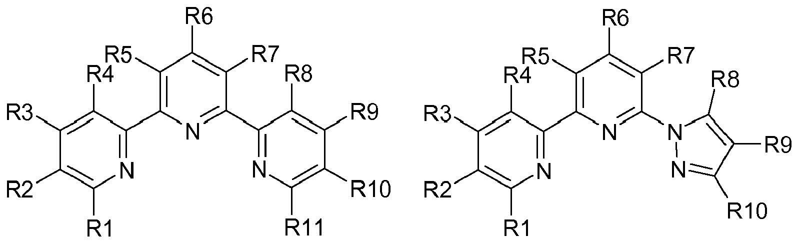 wherein R1, R2, R3, R4, R5, R6, R7, R8, R9, R10 and R11 are independently selected from the group consisting of hydrogen, fluorine, alkyl groups, in particular CH₃, and fluorinated alkyl groups, in particular CF₃; provided at least La comprises at least one fluorine atom.

2. The metal complex according to claim 1, wherein La is selected from the group consisting of the following ligands :

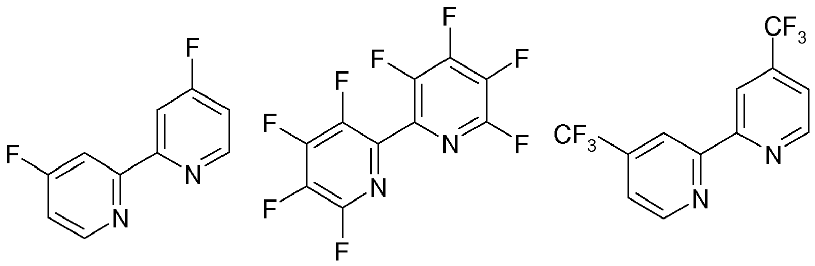

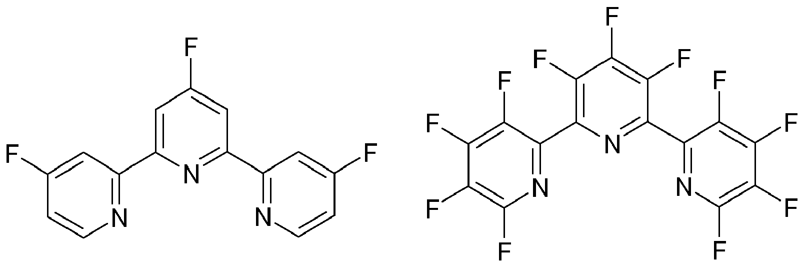

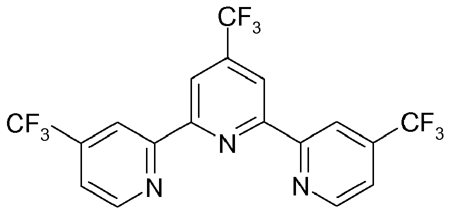

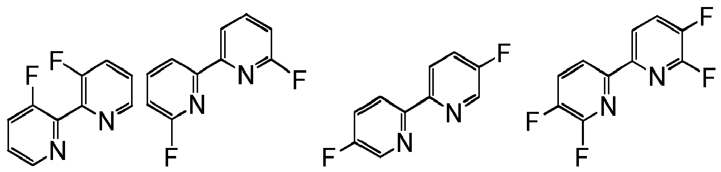

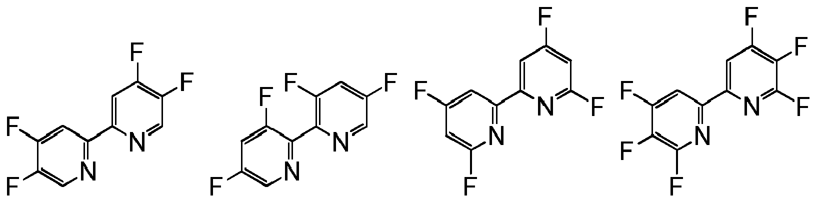

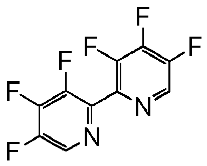

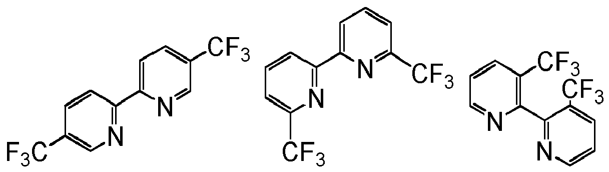

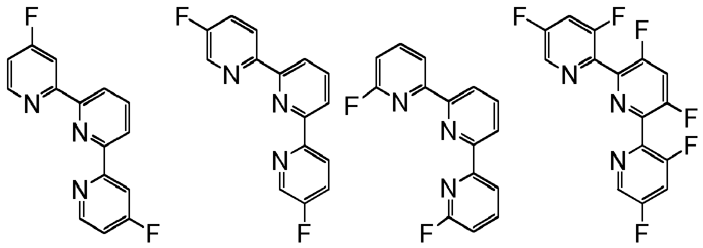

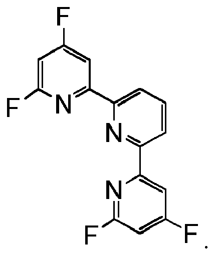

3. The metal complex according to claim 1 or 2, wherein La is selected from the group consisting of the following ligands :

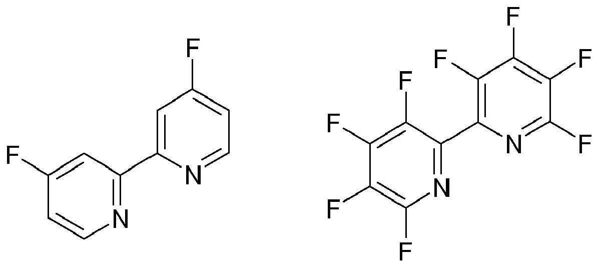

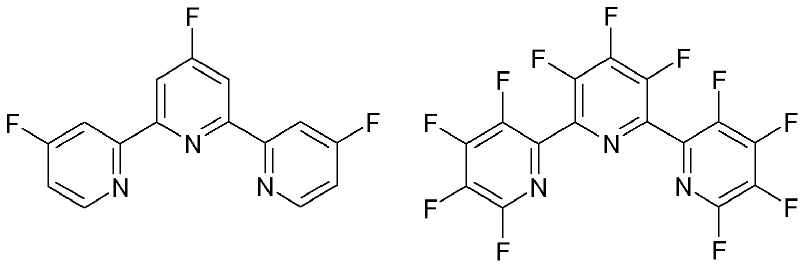

4. The metal complex according to any one of claims 1 to 3, further comprising at least one counter-anion (X).

5. The metal complex according to claim 4, wherein the counter-anion is selected from the group consisting of Cl-, Br-, I-, CN-, NCO-, NCS-, NCSe-, amino groups, phosphate groups, PF6⁻, borates such as [BF₄]⁻, tetracyanoborate ([B(CN)₄]⁻), [B(Ph)₄]⁻, [B(C₆F₅)₄]⁻ bis(trifluoromethanesulfonyl)imide (TFSI⁻), bis(fluorosulfonyl)imide (FSI⁻), and any combination thereof, preferably TFSI⁻.

6. Use of the metal complex according to any one of claims 1 to 5 as redox mediator in photoelectric conversion device, in particular in dye-sensitized solar cell or organic photovoltaic device.

7. A dye-sensitized solar cell comprising the metal complex according to any one of claims 1 to 5 as redox mediator.

8. The dye-sensitized solar cell according to claim 7, further comprising TiOF₂ as semiconductor.

9. A method for preparing the dye-sensitized solar cell according to claim 7 or 8, comprising :
a step of providing an electrode connected to dye-sensitized semiconductor and a counter electrode ; and
a step of providing an electrolyte;
wherein said electrolyte comprises the metal complex according to any one of claims 1 to 5 as redox mediator.

10. Use of cobalt complex having polypyridine ligand comprising at least one fluorine atom, as redox mediator in photoelectric conversion device, in particular in dye-sensitized solar cell.
